# EUROPEAN PATENT SPECIFICATION

(11) **EP 1 685 870 B1**
(45) Date of publication and mention of the grant of the patent: **10.12.2008**
(21) Application number: 06250408.9
(22) Date of filing: 25.01.2006
(51) Int. Cl.: A61M 25/09

(54) **Guidewire with superelastic core**
Führungsdraht mit superelastischem Kern
Fil de guidage à âme superélastique

(30) Priority: 31.01.2005 US 47220
(43) Date of publication of application: 02.08.2006
(73) Proprietor: Cordis Corporation, Miami Lakes, FL 33014 (US)
(72) Inventor: Schreiner, John, Weston Florida 33331 (US)
(74) Representative: Belcher, Simon James

(56) References cited:
- EP-A- 0 868 924
- US-A- 4 538 622
- US-A- 5 402 799
- US-A- 5 404 887
- US-A- 5 749 837
- US-B1- 6 638 267

## Description

The present invention relates to a flexible elongated guidewire which may be used to position a catheter within a patient or may be used in a therapeutic procedure, such as to remove an occlusion within a vessel.

Percutaneous coronary angioplasty (PTA) is a therapeutic medical procedure used to increase blood flow through the coronary artery and can often be used as an alternative to coronary by-pass surgery. An elongated catheter having a deflated balloon at its distal end is guided through a patient's cardiovascular system to the coronary artery of the heart. The balloon is inflated to compress or crack deposits that have accumulated along the inner walls of the coronary artery to widen the artery lumen and increase blood flow.

One prior art technique for positioning the balloon catheter uses an elongated guidewire that is inserted into the patient and passed through the cardiovascular system as guidewire progress is viewed on an x-ray imaging screen. The path the guidewire follows as it is inserted is tortuous. The distal tip is flexible to avoid damaging inner walls of the blood vessels that the guidewire tip contacts along the tortuous path. The distal tip is often pre-bent to a desired configuration so that the guidewire can be inserted into the branching blood vessels along the path. When the tip is pre-bent the physician must be able to orient the tip so it can be pushed into these branching blood vessels.

Representative prior art patents that disclose flexible, elongated guidewires are US-4545390 to Leary; US-4538622 to Samson, et al., US-3906938 to Fleischhacker. The Leary '390 patent discloses a narrow flexible guidewire having a distal portion which tapers and includes a flexible coiled spring at its distal end.

In order to increase the flexibility of guidewires, some guidewires have been formed from a superelastic material, such as Nitinol, which exhibits the property of being extremely flexible, particularly when the Nitinol material becomes warmed as a result of passage through the vasculature of the human body. Representative prior art patents that disclose guidewires formed from a super elastic alloy, such as Nitinol, is US-5069226 to Yamauchi, et al. One disadvantage of guidewires formed from Nitinol is that such guidewires have reduced so-called "torqueability," or the ability to rotate or orient the distal tip of the guidewire by rotating the proximal end of the guidewire.

EP-A-0868924 discusses a guidewire with a shapeable tip. It comprises a corewire preferably made from a superelastic material. The core wire has a uniform diameter portion that tapers to a second uniform diameter at its distal end. A spring surrounds the distal end of the guidewire.

US-5749837 defines the closest prior art and discusses a guidewire that may be a composite in which a distal portion of the core is a super-elastic alloy and the more proximal section is of another material or configuration, e.g. stainless steel wire or rod, stainless steel hypotube, super elastic alloy tubing or carbon-fibre tubing.

The present invention relates to an elongated flexible guidewire designed for insertion into blood vessels to aid in positioning a catheter within the vessel or alternatively, to aid in a therapeutic procedure such as the removal of an obstruction in a vessel.

According to the present invention, there is provided an elongated flexible guidewire as defined in appended claim 1 including a flexible corewire formed from a superelastic material and having a first constant diameter portion that tapers distally along a first tapered portion. A proximal section of the first constant diameter portion has a reduced diameter from that of the first diameter portion. A hypotube extends over the proximal section of the first constant diameter portion and is bonded to the proximal section. In addition, a flexible coil surrounds a portion of the corewire and is attached to the distal tip of the first tapered portion, and a polymer coating covers an outer surface of the guidewire and extends over a major portion of the length of the guidewire.

This guidewire construction results in a flexible distal guidewire portion which can be pre-bent into a desired orientation and easily oriented by the physician while inserting the guidewire into a vessel of the body.

In one embodiment an elongated flexible guidewire is constructed from a flexible corewire having a first constant diameter that extends over a major portion of the guidewire from a proximal end to a distal region of the guidewire. At this distal region, the core tapers uniformly along a first tapered portion to a second lesser constant diameter portion that is shorter than the first constant diameter portion. The corewire then tapers along a second tapered portion in a uniform manner to a final flattened distal portion of the corewire. A flexible coiled wire spring is attached to the corewire along the length of the lesser constant diameter portion and extends distally and separates from the corewire as the corewire tapers along the second tapered portion. At the extreme distal tip of the guidewire, the coiled wire spring is attached to the distal tip of the flattened distal portion of the corewire by, for example brazing, to form the tip of the guidewire. The corewire is preferably formed of a superelastic material, such as Nitinol, which extends for the entire length of the guidewire. In order to increase the "torqueability," or the ability of the distal tip to be oriented by twisting the proximal end of the guidewire, the proximal portion of the Nitinol corewire is ground down to a reduced diameter and a stainless steel hypotube is placed over this portion of the core.

In another embodiment there is provided an elongated flexible guidewire which includes a flexible corewire formed from a superelastic material, such as Nitinol, having a first constant diameter portion that tapers distally along a first tapered portion to a second lesser constant diameter portion shorter than the first diameter portion and that again tapers distally along a second tapered portion to a flattened distal portion of the guidewire. The first constant diameter portion includes a proximal section having a reduced diameter section. A hypotube extends over the reduced diameter section of the first constant diameter section and is bonded to the reduced diameter section. In addition, a flexible coil surrounds the corewire and is attached to the corewire along a length of the second lesser constant diameter portion of the corewire and is also attached to a distal end of the flattened distal portion of the corewire. Preferably, a polymer coating is applied to the outer surface of the guidewire and extends over a major portion of the guidewire.

In accordance with another aspect of the present invention, the hypotube is formed of a flexible material but a material which has excellent torque characteristics, such as stainless steel. The hypotube preferably extends over the corewire from the proximal end of the corewire for a length of at least about half the length of the corewire in order to in part improve torque characteristic to the corewire which is formed of a superelastic material, such as Nitinol.

Embodiments of the invention will now be described by way of example with reference to the accompanying drawings, in which:
Figure 1 is a diagrammatic view showing a blood vessel that has been occluded with deposits along an inner wall and illustrating the positioning of a flexible guidewire within a blood vessel;
Figure 2 is partially sectioned, elevation segmented view of a flexible guidewire constructed in accordance with the invention; and
Figure 3 is an enlarged sectioned view as seen from the plane defined by the lines 3-3 in Figure 2.

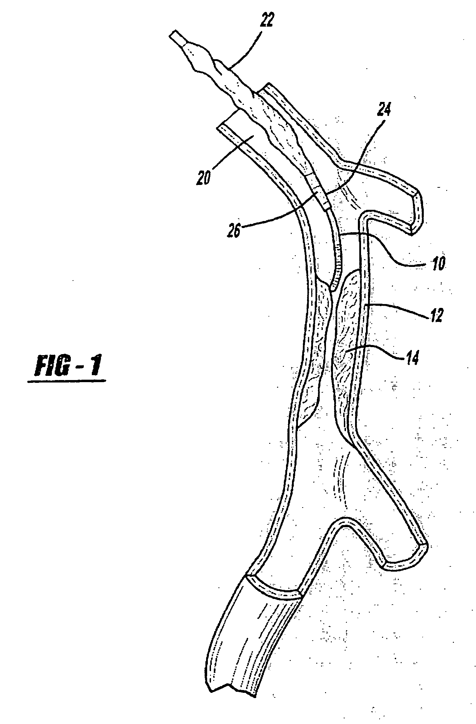

Turning now to the drawings, Figure 1 illustrates a distal portion of a flexible, small diameter guidewire 10 that can be guided through a patient's vascular system. A distal end of the guidewire is approaching a region in a blood vessel 12 having an occlusion 14 which has restricted blood flow through the blood vessel 12. The guidewire 10 is long enough to be routed from an entry point of the patient through the vessels of the patient to the obstructed blood vessel region. As the guidewire 10 is inserted along the tortuous path to the obstructed blood vessel region, an attending physician conducting the procedure monitors progress of the guidewire 10 on a fluorographic viewing screen.

The Figure 1 depiction illustrates use of a guidewire for routing a balloon catheter 20 to the vicinity of the occlusion 14. The balloon catheter 20 includes a first passageway or lumen which extends from a proximal location outside the patient's body to a distally located balloon 22. A distal tip portion 24 of the catheter 20 includes a marker band 26 to aid the attending physician in monitoring balloon catheter progress as it is positioned within the patient. A second, center passageway or lumen in the catheter 20 has a diameter sufficient to accommodate the guidewire 10 so that once the guidewire is properly positioned the catheter 20 can be slid over the guidewire to a desired location.

The distal tip portion of the guidewire 10 is flexible and can be bent to a predetermined configuration to facilitate routing the guidewire 10 along the vascular system. The pre-bent tip can be oriented by the physician. Torque applied to the proximal end of the guidewire is transmitted along the length of the guidewire to orient or rotate the distal tip of the guidewire in order to direct the distal tip in a desired direction.

In use, a distal end of the guidewire 10 is routed through a narrow passageway in the occlusion 14 and the balloon catheter 20 slipped over the guidewire until the balloon 22 bridges the occlusion 14 within the blood vessel 12. The balloon 22 is then pressurized from a pressure source and as the balloon outer surface contacts the occlusion 14, inner walls of the obstruction are compressed and a wider lumen or passageway is created in the blood vessel 12.

Although the Figure 1 depiction has been used to illustrate one use of the guidewire, it should be appreciated that a guidewire constructed in accordance with the invention has utility with angiographic catheters or any application requiring the routing of a tubular device within a patient, or alternatively, may be used with certain therapeutic procedures, such as the removal of an obstruction within a vessel.

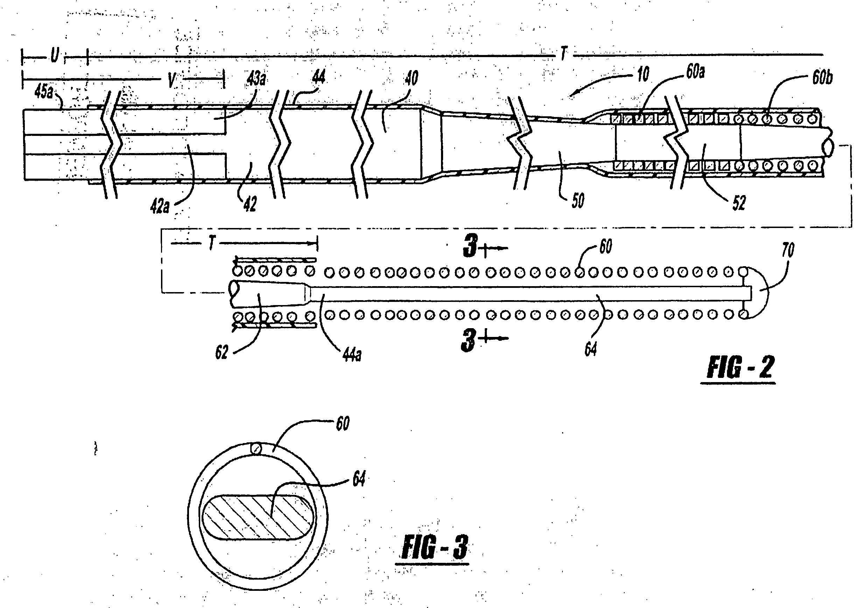

Turning now to Figure 2, the guidewire 10 includes a corewire 40 formed from a superelastic material, such as Nitinol, having a first uniform diameter proximal portion 42 extending well over half the length of the guidewire. To increase the "torqueability," or torque characteristics of the guidewire 10, the proximal portion 42a of the uniform diameter portion 42 is ground down to a reduced diameter and a stainless steel hypotube 43a is placed over the reduced diameter portion of the proximal portion 42a and is bonded to the proximal portion 42a by use of an adhesive, such as epoxy. The proximal portion 42a of the uniform diameter portion of the corewire 40 extends for a length "V" which is preferably about 120 cm.

Preferably, the total length of the guidewire 10 is approximately 150 centimeters. The outer surface of a most proximal segment 45a of the guidewire having a length indicated as "U" is not covered with a lubricious coating, but the remaining length "T" of the guidewire 10 up to a distal tip portion 44a is covered with a thin Teflon coating 44. The exposed segment 45a may be more easily grasped by the attending physician in order to rotate the proximal end of the guidewire 10.

The Teflon coating which is applied to the guidewire 10 preferably has a thickness of approximately 0.016 mm (0.00065 inch) and is applied by a hot dipping process. The corewire 40 tapers along a portion 50 in a uniform manner to a second reduced constant diameter portion 52. The reduced constant diameter portion 52 is bounded by a coiled wire spring 60. The proximal portion 60a of the spring 60 is comprised of coil turns having a rectangular cross-section and the distal portion 60b of the spring 60 is comprised of coil turns having a circular cross-section.

The spring 60 separates from the corewire 40 where the core begins to taper in a uniform manner along a portion 62. A distal portion 64 of the corewire 40 is flattened and surrounded by the less tightly coiled portion of the spring 60. This distal portion of the guidewire 10 may be pre-bent to a particular configuration by the attending physician to facilitate insertion of the guidewire within the vessels of a patient.

At the extreme distal tip portion of the guidewire 10, braze material 70 is used to attach the distal portion of the spring 60 to the flattened portion 64 of the corewire 40. A preferred braze material is a gold alloy which upon being applied defines a hemispherical bead which covers several coils and is polished to a smooth shape so that it does not damage the inner lining of the blood vessels as the tip comes in contact with those linings.

The dimensions shown are for a preferred embodiment in the invention for use in small diameter blood vessels. These dimensions are representative of this use and are not intended to limit the invention, but rather define a small diameter guidewire whose characteristics are particularly advantageous.

## Claims

1. An elongated flexible guidewire (10) having a proximal end, the guidewire (10) comprising:
a flexible corewire (40) formed from a superelastic material and having a first constant diameter portion (42) that tapers distally along a first tapered portion (50), a proximal section (42a) of said first constant diameter portion (42) having a reduced diameter section from that of said first diameter portion;
a hypotube (43a) extending over said proximal section (42a) of said first constant diameter portion (42) and being bonded to said proximal section (42a);
a flexible coil (60) surrounding a portion of the corewire (40) and attached to a distal tip of said first tapered portion (50); and,
a polymer coating (44) covering an outer surface of said guidewire (10) extending over a major portion of the guidewire (10);
**characterised in that** the proximal section (42a) extends-from said proximal end of said guidewire (10),

2. An elongated flexible guidewire (10) according to claim 1 wherein:
the first constant diameter portion (42) of the flexible corewire (40) tapers distally along a first tapered portion (50) to a second lesser constant diameter portion (52) shorter than said first diameter portion (42) and that again tapers distally along a second tapered portion (62) to a flattened distal portion (64) of said corewire (40); and the flexible coil (60) surrounding the corewire (40) is attached to the corewire (40) along a length of the second lesser constant diameter portion (52) of the corewire (40) and attached to a distal end of the flattened distal portion (64) of the corewire (40).

3. An elongated flexible guidewire (10) as defined in Claim 2, wherein the coil (60) is attached to the distal end of the flattened distal portion (64) of the corewire (40) with a brazing material (70) which forms a rounded distal tip of the guidewire.

4. An elongated flexible guidewire (10) as defined in Claim 3, wherein an outer diameter of said hypotube (43a) is approximately equal to a diameter of the first constant diameter portion (42).

5. An elongated flexible guidewire (10) as defined in Claim 1 or 2, wherein said hypotube (43a) is formed of stainless steel.

6. An elongated flexible guidewire (10) as defined in Claim 5, wherein said hypotube (43a) is bonded to said reduced diameter section (42a) with an adhesive material.

7. An elongated flexible guidewire (10) as defined in Claim 6, wherein said adhesive material is an epoxy.

8. An elongated flexible guidewire (10) as defined in Claim 1 or 2, wherein said hypotube (43a) extends from the proximal section of the guidewire for at least one-half the length of the guidewire.

## Patentansprüche

1. Länglicher flexibler Führungsdraht (10) mit einem proximalen Ende, wobei der Führungsdraht (10) Folgendes umfasst:
einen flexiblen Kerndraht (40), der aus einem superelastischen Material gebildet ist und einen ersten Teil mit konstantem Durchmesser (42) aufweist, der sich distal entlang eines ersten sich verjüngenden Teils (50) verjüngt, wobei ein proximaler Abschnitt (42a) des ersten Teils mit konstantem Durchmesser (42) einen reduzierten Durchmesserabschnitt von dem des ersten Durchmesserteils aufweist;
eine Hyporöhre (43a), die sich über den proximalen Abschnitt (42a) des ersten Teils mit konstantem Durchmesser (42) erstreckt und mit dem zweiten proximalen Abschnitt (42a) verbunden ist;
eine flexible Spule (60), die einen Teil des Kemdrahtes (40) umgibt, und an einer distalen Spitze des ersten sich verjüngenden Teils (50) angebracht ist; und
eine Polymerbeschichtung (44), die eine äußere Oberfläche des Führungsdrahtes (10) bedeckt und sich über einen Hauptteil des Führungsdrahtes (10) erstreckt;
**dadurch gekennzeichnet, dass** der proximale Abschnitt (42a) sich von dem proximalen Ende zu dem Führungsdraht (10) erstreckt.

2. Länglicher flexibler Führungsdraht (10) nach Anspruch 1, bei welchem der erste Teil mit konstantem Durchmesser (42) des flexiblen Kerndrahtes (40) sich distal entlang des ersten sich verjüngenden Teils (50) zu einem zweiten Teil mit geringerem konstanten Durchmesser (52) verjüngt, das kürzer als das erste Durchmesserteil (42) ist und das sich wiederum distal entlang eines zweiten sich verjüngenden Teils (42) verjüngt zu einem zweiten abgeflachten distalen Teil (64) des Kemdrahtes (40); und die flexible Spule (60), welche den Kerndraht (40) umgibt, an dem Kerndraht (40) entlang einer Länge des zweiten Teils mit geringerem konstanten Durchmesser (52) des Kemdrahtes (40) angebracht ist und an dem distalen Ende des abgeflachten distalen Teils (64) des Kerndrahtes (40) angebracht ist.

3. Länglicher flexibler Führungsdraht (10) nach Anspruch 2, bei welchem die Spule (60) an dem distalen Ende des abgeflachten distalen Teils (64) des Kemdrahtes (40) angebracht ist mit einem Lötmaterial (70), welches eine runde distale Spitze des Führungsdrahtes bildet.

4. Länglicher flexibler Führungsdraht (10) nach Anspruch 3, bei welchem ein äußerer Durchmesser der Hyporöhre (43a) etwa gleich zu einem Durchmesser des ersten Teils mit konstantem Durchmesser (42) ist.

5. Länglicher flexibler Führungsdraht (10) nach Anspruch 1 oder 2, bei welchem die Hyporöhre (43a) aus einem Edelstahl gebildet ist.

6. Länglicher flexibler Führungsdraht (10) nach Anspruch 5, bei welchem die Hyporöhre (43a) mit dem Abschnitt reduzierten Durchmessers (42a) durch einen Klebstoff verbunden ist.

7. Länglicher flexibler Führungsdraht (10) nach Anspruch 6, bei welchem der Klebstoff ein Epoxid ist.

8. Länglicher flexibler Führungsdraht (10) nach Anspruch 1 oder 2, bei welchem die Hyporöhre (43a) sich von dem proximalen Abschnitt des Führungsdrahtes über wenigstens die halbe Länge des Führungsdrahtes erstreckt.

## Revendications

1. Fil de guidage flexible allongé (10) ayant une extrémité proximale, le fil de guidage (10) comprenant :
■ une âme flexible (40) constituée d'un matériau super-élastique et ayant une première partie à diamètre constant (42) qui s'affine distalement le long d'une première partie affilée (50), une partie proximale (42a) de ladite première partie à diamètre constant (42) ayant une partie à diamètre réduit par rapport à ladite première partie de diamètre ;
■ un hypotube (43a) s'étendant sur ladite section proximale (42a) de ladite première partie à diamètre constant (42) et étant relié à ladite partie proximale (42a) ;
■ une spirale flexible (60) entourant une partie de l'âme (40) et fixée à une extrémité distale de ladite première partie effilée (50) ; et
■ un revêtement polymère (44) couvrant une surface externe dudit fil de guidage (10) s'étendant sur une partie majeure du fil de guidage (10) ;
**caractérisé en ce que** la partie proximale (42a) s'étend de ladite extrémité proximale dudit fil de guidage (10).

2. Fil de guidage flexible allongé (10) selon la revendication 1, dans lequel la première partie à diamètre constant (42) de l'âme flexible (40) s'affine distalement le long d'une première partie effilée (50) jusqu'à une deuxième partie à diamètre moins constant (52) plus courte que ladite première partie de diamètre (42) et qui s'affine à nouveau distalement le long d'une deuxième partie effilée (62) jusqu'à une partie distale aplatie (64) de ladite âme (40) ; et la spirale flexible (60) entourant l'âme (40) étant fixée à l'âme (40) le long d'une longueur de la deuxième partie à diamètre moins constant (52) de l'âme (40) et fixée à une extrémité distale aplatie (64) de l'âme (40).

3. Fil de guidage flexible allongé (10) tel que défini à la revendication (2), dans lequel la spirale (60) est fixée à l'extrémité distale de la partie distale aplatie (64) de l'âme (40) avec un matériau de brasage (70) qui forme une extrémité distale arrondie du fil de guidage.

4. Fil de guidage allongé (10) tel que défini à la revendication 3, dans lequel un diamètre externe dudit hypotube (43a) est à peu près égal au diamètre de la première partie à diamètre constant (42).

5. Fil de guidage allongé (10) selon la revendication 1 ou 2, dans lequel ledit hypotube (43a) est constitué d'acier inoxydable.

6. Fil de guidage flexible allongé (10) tel que défini à la revendication 5, dans lequel ledit hypotube (43a) est relié à ladite partie à diamètre réduit (42a) avec un matériau adhésif.

7. Fil de guidage flexible allongé (10) selon la revendication 6, dans lequel ledit matériau adhésif est un époxy.

8. Fil de guidage flexible allongé (10) tel que défini à la revendication 1 ou 2, dans lequel ledit hypotube (43a) s'étend de la partie proximale du fil de guidage sur au moins une demi-longueur du fil de guidage.
